# EUROPEAN PATENT APPLICATION

(11) **EP 4 585 685 A1**
(43) Date of publication of application: **16.07.2025**
(21) Application number: 23863409.1
(22) Date of filing: 31.08.2023
(51) Int. Cl.: C12N 15/11, C12N 15/63, C12Q 1/6809, A61K 39/00

(54) **CONSTRUCT COMPRISING UTR SEQUENCE THAT IMPROVES INTRACELLULAR STABILITY AND BIOSYNTHESIS OF MRNA AND USE THEREOF**

(30) Priority: 08.09.2022 KR 20220114207
(71) Applicant: Theragen Bio Co., Ltd., Gyeonggi-do 13488 (KR)
(72) Inventor: PAIK, Soon Myung, Seongnam-si, Gyeonggi-do 13488 (KR); KIM, Hae Suk, Seongnam-si, Gyeonggi-do 13488 (KR); HONG, Seong Eui, Seongnam-si, Gyeonggi-do 13488 (KR); KIM, Seong Gwang, Seongnam-si, Gyeonggi-do 13488 (KR); HEO, Dong Hyuk, Seongnam-si, Gyeonggi-do 13488 (KR); JO, Eun Bi, Seongnam-si, Gyeonggi-do 13488 (KR)
(74) Representative: Müller Schupfner & Partner Patent- und Rechtsanwaltspartnerschaft mbB (Muc)
(86) International application number: PCT/KR2023/012989
(87) International publication number: WO 2024/053933

(57) **Abstract**

Unlike DNA, mRNA has the advantage of enabling transient expression of a desired protein directly in the cytoplasm without having to enter the nucleus, and thus, attempts to use mRNA vaccines are actively underway. However, mRNA has lower structural stability than DNA, and thus has limitations in industrial application. Therefore, the present invention relates to a UTR sequence that improves the intracellular stability and biosynthesis of mRNA, and a composition for stabilizing nucleic acids, comprising the UTR sequence. The UTR sequence of the present invention has excellent effects of stabilizing nucleic acids and promoting the expression of a target protein, and thus is expected to be greatly used in the health/medical field.

## Description

### [Technical Field]

The present invention relates to a sequence for stabilizing nucleic acids, particularly, an untranslated region (UTR) sequence that improves the intracellular stability and biosynthesis of mRNA, and a composition for stabilizing nucleic acids comprising the UTR sequence.

### [Background Art]

Unlike DNA, mRNA has the advantage of enabling transient expression of a desired protein directly in the cytoplasm without having to enter the nucleus, and thus is attracting attention as a new treatment method in the health/medical field. In particular, attempts to use mRNA vaccines are actively underway for treatment and prevention of diseases based on global biotech and pharmaceutical companies, but mRNA has lower structural stability than DNA, and thus has limitations in industrial application due to lower structural stability than DNA. Therefore, recently, to solve these problems, attempts have been made to increase the intracellular stability of mRNA and improve the biosynthetic efficiency of protein by using the UTR. The UTR plays an auxiliary role in enabling the effective synthesis of peptides or proteins to be therapeutic targets, and is independent of a sequence of a gene to be synthesized to enable the expression of various peptides or proteins. Particularly, in the mRNA vaccine field, the UTR improves the stability of mRNA vaccines and enables efficient synthesis of antigens (neoantigens in the case of cancer vaccines) *in vivo,* so that even a small dose of vaccine can be expected to be highly effective in treating and preventing diseases.

Accordingly, the present invention relates to a sequence for stabilizing nucleic acids, particularly, an untranslated region (UTR) sequence that improves the intracellular stability and biosynthesis of mRNA, and a composition for stabilizing nucleic acids including the UTR sequence. The UTR sequence of the present invention has excellent effects of stabilizing nucleic acids and promoting the expression of a target protein, and thus is expected to be greatly used in the health/medical field.

### [Detailed Description of the Invention]

### [Technical Problem]

An object of the present invention is to provide a sequence for stabilizing nucleic acids, particularly, an untranslated region (UTR) sequence that improves the intracellular stability and biosynthesis of mRNA, and a selection method thereof.

Another object of the present invention is to provide an artificial nucleic acid including a sequence for stabilizing nucleic acids, particularly, an untranslated region (UTR) sequence that improves the intracellular stability and biosynthesis of mRNA, and a manufacturing method thereof.

Yet another object of the present invention is to provide a vector including the artificial nucleic acid and a manufacturing method thereof.

Still another object of the present invention is to provide a transfectant transfected with the vector including the artificial nucleic acid, and a manufacturing method thereof.

Still another object of the present invention is to provide a composition for stabilizing nucleic acids including the artificial nucleic acid, and a manufacturing method thereof.

Still another object of the present invention is to provide an immunity-boosting composition including the artificial nucleic acid, and a manufacturing method thereof.

Still another object of the present invention is to provide a kit for detecting nucleic acids including the artificial nucleic acid, and a manufacturing method thereof.

Still another object of the present invention is to provide a pharmaceutical composition for preventing or treating diseases including the artificial nucleic acid, and a manufacturing method thereof.

However, technical objects of the present invention are not limited to the aforementioned purpose and other objects which are not mentioned may be clearly understood to those skilled in the art from the following description.

### [Technical Solution]

Hereinafter, various embodiments described herein are described with reference to the drawings. In the following description, various specific details are set forth, such as specific forms, compositions, and processes, in order to provide a thorough understanding of the present invention. However, particular embodiments may be implemented with one or more of these specific details, or with other known methods and formats. In other examples, known processes and manufacturing techniques are not described as specific details so as not to make the present invention unnecessarily obscure. References throughout this specification to "one embodiment" or "an embodiment" mean that particular features, shapes, compositions or characteristics described in connection with the embodiment are included in one or more embodiments of the present invention. Accordingly, the details of "one embodiment" or "an embodiment" expressed in various locations throughout this specification do not necessarily refer to the same embodiment of the present invention. Additionally, the particular features, shapes, compositions, or characteristics may be combined in any suitable manner in one or more embodiments.

Unless otherwise specifically defined in the specification, all scientific and technical terms used herein have the same meaning as commonly understood by those skilled in the art to which the present invention pertains.

As used herein, the term "untranslated region (UTR)" means a portion of a nucleic acid molecule that is not translated into a protein. The untranslated region located in a 5' direction of the nucleic acid is called 5'-UTR, and the untranslated region located in a 3' direction of the nucleic acid is called 3'-UTR. In detail, the 5'-UTR is located 5' (i.e. "upstream") of an open reading frame and is typically understood as a specific section of messenger RNA (mRNA) located 5' of the open reading frame of mRNA. Preferably, the 5'-UTR has a length of 10 to 100, 20 to 100, 20 to 80, 20 to 60, 20 to 50, 20 to 40, or 20 to 30 nucleotides (nt). The 5'-UTR may include an element for regulating gene expression, so-called a regulatory element, but is not limited thereto, and the regulatory element may be a ribosome binding site. The 5'-UTR may be modified after transcription through the addition of 5'-CAP. The 5'-UTR of mRNA is not translated into an amino acid sequence, and the 5'-UTR sequence is generally encoded by a gene that is transcribed into each mRNA during the gene expression process. The genomic sequence may be modified by a process such as 5'-capping or splicing after transcription by including an alternative intron. The 5'-UTR is a nucleotide proximal to the 5'-CAP of the modified mRNA, and typically corresponds to a transcription start site. Herein, "corresponding" means that the 5'-UTR sequence may be an RNA sequence, such as an mRNA sequence, or a DNA sequence corresponding to such an RNA sequence. The 3'-UTR is located 3' (i.e. "downstream") of an open reading frame and is typically understood as a specific section of messenger RNA (mRNA) located 3' of the open reading frame of mRNA. Preferably, the 3'-UTR has a length of 10 to 1000, 10 to 500, 20 to 500, 20 to 300, 20 to 200, 20 to 100, 20 to 80, 20 to 60, 20 to 50, 20 to 40, or 20 to 30 nucleotides (nt). The 3'-UTR is located between a protein-coding region of the mRNA and a poly A sequence. The 3'-UTR sequence is generally encoded by a gene that is transcribed into each mRNA during the gene expression process, and the genomic sequence may be modified by a process such as splicing or 3'-polyadenylation, after transcription by including an alternative intron. The 3'-UTR is a nucleotide proximal to the poly A sequence of the modified mRNA, and typically corresponds to a transcription termination site. Herein, "corresponding" means that the 3'-UTR sequence may be an RNA sequence, such as an mRNA sequence, or a DNA sequence corresponding to such an RNA sequence.

As used herein, the term "nucleic acid" is a concept including deoxyribonucleic acid (DNA) and ribonucleic acid (RNA). The "nucleic acid" is used synonymously with the term "polynucleotide". Preferably, the nucleic acid is a polymer formed by including nucleotide monomers covalently bonded to each other by phosphodiester bonds of a sugar/phosphate backbone. Alternatively, the nucleic acid includes DNA or RNA that is base-modified, sugar-modified, or backbone-modified.

As used herein, the term "stabilizing nucleic acid" is a concept of stabilizing a nucleic acid structure by preventing the collapse or modification of a nucleic acid structure inside or outside a cell. In general, RNA is composed of a single strand and has higher activity than DNA to have lower structural stability. Therefore, the stabilizing of the nucleic acid in the present invention is to prevent the structural collapse or modification of DNA or RNA, more preferably RNA, and much more preferably mRNA, and sequences represented by SEQ ID NO: 1 to SEQ ID NO: 18, and SEQ ID NO: 20 to SEQ ID NO: 37 of the present invention were each evaluated to have an excellent nucleic acid stabilization effect.

As used herein, the term sequence homology refers to the percentage indicating the degree to which two sequences to be compared are identical. In order to determine the degree of homology, it is preferable that the sequences to be compared have the same length, but if the sequence lengths are different, the degree of homology is calculated based on a longer sequence among the sequences to be compared. For example, a sequence of 10 nt has 80% homology with an 8 nt sequence that is represented by the same sequence as a part thereof.

In the present invention, the artificial nucleic acid having the excellent nucleic acid stabilization effect of the present invention may be an artificial nucleic acid including a sequence having 70% or more homology with any one selected from the group consisting of SEQ ID NO: 1 to SEQ ID NO: 18, and SEQ ID NO: 20 to SEQ ID NO: 37. Alternatively, the artificial nucleic acid of the present invention may be an artificial nucleic acid including a sequence having 80% or more homology with any one selected from the group consisting of SEQ ID NO: 1 to SEQ ID NO: 18, and SEQ ID NO: 20 to SEQ ID NO: 37. Alternatively, the artificial nucleic acid of the present invention may be an artificial nucleic acid including a sequence having 90% or more homology with any one selected from the group consisting of SEQ ID NO: 1 to SEQ ID NO: 18, and SEQ ID NO: 20 to SEQ ID NO: 37. Alternatively, the artificial nucleic acid of the present invention may be an artificial nucleic acid including a sequence having 95% or more homology with any one selected from the group consisting of SEQ ID NO: 1 to SEQ ID NO: 18, and SEQ ID NO: 20 to SEQ ID NO: 37. Alternatively, the artificial nucleic acid of the present invention may be an artificial nucleic acid including a sequence having 100% homology with any one selected from the group consisting of SEQ ID NO: 1 to SEQ ID NO: 18, and SEQ ID NO: 20 to SEQ ID NO: 37.

As used herein, the term "artificial nucleic acid" may be understood as a non-natural nucleic acid molecule that does not exist in nature. The artificial nucleic acid may be 100% composed of a sequence that does not exist in nature, or may be composed by mixing a part of a sequence that exists in nature (wild type) and a part of a sequence that does not exist in nature. In the case where the sequence is composed by mixing a part of the sequence existing in nature and a part of the sequence not existing in nature, the sequence existing in nature may be a mixture of sequences derived from one or more species. The artificial nucleic acid may be non-natural due to a non-naturally occurring modification of its individual sequences, for example, a structural modification of a nucleotide that does not occur naturally. In addition, the artificial nucleic acid may be a DNA molecule, an RNA molecule, or a hybrid molecule including DNA and RNA portions.

As used herein, the term "vector" may be understood as a type of the artificial nucleic acid. In the context of the present invention, the vector is a concept that includes a sequence having at least 70% homology with an artificial nucleic acid selected for stabilization of nucleic acids, specifically, any one selected from the group consisting of SEQ ID NO: 1 to SEQ ID NO: 18, and SEQ ID NO: 20 to SEQ ID NO: 37. Such a vector may be a storage vector, an expression vector, a cloning vector, a transfer vector, etc. The storage vector is a vector which allows for the convenient storage of nucleic acid molecules, for example, mRNA molecules. The expression vector may be used for the production of RNA, for example, mRNA, or expression products, such as peptides, polypeptides or proteins. The cloning vector is a vector which typically includes a cloning site which may be used to incorporate a nucleic acid sequence into the vector. The cloning vector may be, for example, a plasmid vector or a bacteriophage vector. The transfer vector may be a vector suitable for transferring nucleic acid molecules into cells or organisms, for example, a viral vector. In the context of the present invention, the vector may be, for example, an RNA vector or a DNA vector. Preferably, the vector is a DNA molecule. Preferably, the vector of the present invention includes an artificial nucleic acid selected for stabilization of nucleic acids, specifically a sequence having 70% or more homology with any one selected from the group consisting of SEQ ID NO: 1 to SEQ ID NO: 18, and SEQ ID NO: 20 to SEQ ID NO: 37 and a sequence of a target gene (a sequence encoding a target protein) for the purpose of expression regulation. The vector is preferably a plasmid vector, but is not limited thereto.

As used herein, the term "transfection" means introducing a nucleic acid molecule such as a DNA or RNA (e.g., mRNA) molecule into a cell, preferably a eukaryotic cell, to change an original phenotype of a subject to be introduced. The transfection may be performed by any method known to those skilled in the art for introducing a nucleic acid molecule into a cell, preferably a eukaryotic cell such as a mammalian cell. Such a method includes, for example, electroporation, lipofection based on cationic lipids and/or liposomes, calcium phosphate precipitation, nanoparticle-based transfection, virus-based transfection, or cationic polymer-based transfection such as DEAE-dextran or polyethyleneimine. Alternatively, the transfection may be performed using a virus, for example, a lentivirus. A subject to be introduced whose original phenotype has been changed by being transfected by the method is called a transfectant.

As used herein, the term "immunity-boosting" means that a desired immune response is amplified against a selected antigen, such as a characteristic component of a bacterial surface, a viral particle, a tumor antigen, etc. The immunity-boosting may be naturally or artificially induced to amplify the immune responses, and in the case of artificial immune response amplification, vaccine administration is the most common method. A vaccine is typically understood as a prophylactic or therapeutic substance that provides at least one antigen, preferably an immunogen. The antigen or immunogen may be derived from any substance suitable for vaccination. For example, the antigen or immunogen may be derived from bacterial or viral particles, or the like, or from a tumor or cancer tissue. The antigen or immunogen stimulates an adaptive immune system *in vivo.* In the related art, vaccines using proteins or peptides derived from antigens or immunogens as stimulators of the adaptive immune system *in vivo* were dominated, but attempts to use nucleic acids as stimulators have recently continued. Particularly, unlike DNA, mRNA has the advantage of enabling transient expression of a desired protein directly in the cytoplasm without having to enter the nucleus, and thus is attracting attention as a new treatment method in the health/medical field. However, mRNA has lower structural stability than DNA, and thus has limitations in industrial application. Therefore, recently, to solve these problems, attempts have been made to increase the intracellular stability of mRNA and improve the biosynthetic efficiency of protein by using the UTR. In this context, any one sequence selected from the group consisting of SEQ ID NO: 1 to SEQ ID NO: 18, and SEQ ID NO: 20 to SEQ ID NO: 37 having an excellent nucleic acid stabilization effect of the present invention, or a sequence having 70% or more homology therewith may be administered as a vaccine together with a nucleic acid derived from an antigen or immunogen suitable for desired immune-boosting. In this case, any one sequence selected from the group consisting of SEQ ID NO: 1 to SEQ ID NO: 18, and SEQ ID NO: 20 to SEQ ID NO: 37of the present invention, or a sequence having 70% or more homology therewith is preferably located before/after antigen genetic information in the nucleic acid vaccine, and the nucleic acid vaccine is expected to have the effects of protecting the nucleic acid vaccine from degradation enzymes *in vivo* and boosting the nucleic acid sequence as an antigen to be effectively translated. In addition, since the stability of the nucleic acid vaccine is improved and the antigen may be efficiently synthesized *in vivo,* a high effect in disease treatment and prevention can be expected even with a small amount of vaccine administration. In addition, since the dose of the vaccine to be administered to meet an expected therapeutic effect is reduced, an effect of lowering vaccine production cost may be expected. The vaccine may additionally include an adjuvant (component) to boost the expected effect. The adjuvant is typically a pharmaceutical and/or immunological agent capable of modifying or enhancing the effect of another agent, such as a drug or vaccine. This adjuvant is interpreted in a broad sense and represents a wide range of substances. Typically, these substances may increase the immunogenicity of the antigen. For example, the adjuvant may be recognized by an innate immune system and may induce an innate immune response. The vaccine of the present invention, which includes a sequence having at least 70% homology with any one selected from the group consisting of SEQ ID NO: 1 to SEQ ID NO: 18, and SEQ ID NO: 20 to SEQ ID NO: 37, and a nucleic acid derived from an antigen or immunogen suitable for desired immunity-boosting, may be indefinitely expanded in the use depending on a type of antigen or immunogen to be included. For example, when a nucleic acid derived from cancer tissue is included as the antigen or immunogen, the vaccine is a cancer vaccine. The cancer vaccine herein may mean an active immunotherapy that activates the immune system by administering a cancer-specific antigen of cancer cells to a cancer patient to activate the immune function *in vivo,* thereby attacking cancer cells and removing cancer. When the vaccine (or vaccine composition) of the present invention is used as the cancer vaccine, the nucleic acid derived from the cancer tissue is preferably a neoantigen (a specific antigen produced only by cancer cells) nucleic acid, but is not limited thereto. The cancer may refer to a disease that forms a mass or tumor composed of undifferentiated cells that proliferate indefinitely within a tissue regardless of order. The cancer may mean that abnormal cells to be killed proliferate excessively and, in some cases, invade surrounding tissues and organs to form a mass and destroy or transform existing structures. Ultimately, the cancer may refer to a general term for a group of diseases that may infiltrate and destroy surrounding normal tissues or organs and metastasize from the primary lesion to any organ of a subject to create a new growth site, thereby taking the life of the subject. In addition, the cancer may be any one selected from the group consisting of oral cancer, liver cancer, stomach cancer, colon cancer, breast cancer, lung cancer, bone cancer, pancreatic cancer, skin cancer, head cancer, cervical cancer, skin cancer, melanoma, cervical cancer, ovarian cancer, colorectal cancer, small intestine cancer, rectal cancer, fallopian tube cancer, anal cancer, endometrial carcinoma, vaginal carcinoma, vulvar carcinoma, Hodgkin's disease, esophageal cancer, lymphoma, bladder cancer, gallbladder cancer, endocrine cancer, thyroid cancer, parathyroid cancer, adrenal cancer, soft tissue sarcoma, urethral cancer, penile cancer, prostate cancer, chronic leukemia, acute leukemia, lymphocytic lymphoma, kidney cancer, ureteral cancer, renal cell carcinoma, renal pelvic carcinoma, central nervous system tumor, primary central nervous system lymphoma, spinal cord tumor, brainstem glioma, and pituitary adenoma, but is not limited thereto.

As used herein, the term "markers" mean indicators that may detect changes in the body using proteins, DNA, RNA, metabolites, etc. Specifically, tumor markers (TMs) or cancer cell markers are substances that indicate the presence of cancer cells, and mean that examining for the substance in blood, tissue, or excrement is helpful as an indicator for the diagnosis or treatment of cancer among substances produced by cancer cells or substances produced when normal cells in the body react with cancer cells. For example, the markers include carcinoembryonic antigen (CEA), which is a type of glycoprotein, as a pan-tumor marker that significantly increases in colon cancer, pancreatic cancer, stomach cancer, breast cancer, etc., alfa-fetoprotein (AFP) as a marker of primary liver cancer, CA 19-9 as a marker of pancreatic cancer and bile duct cancer, CA 125 as a marker of ovarian cancer, and prostate specific antigen (PSA) as a marker of prostate cancer. In the case where the artificial nucleic acid selected for stabilization of nucleic acids of the present invention, specifically, the sequence having 70% or more homology with any one selected from the group consisting of SEQ ID NO: 1 to SEQ ID NO: 18, and SEQ ID NO: 20 to SEQ ID NO: 37, is used as a marker composition for diagnosing or treating a specific disease, the disease may be cancer, but is not limited thereto. The artificial nucleic acid including the sequence having 70% or more homology with any one selected from the group consisting of SEQ ID NO: 1 to SEQ ID NO: 18, and SEQ ID NO: 20 to SEQ ID NO: 37 of the present invention may be used as a cancer cell marker composition together with an antibody suitable for a cancer cell marker or a nucleic acid sequence complementary to a nucleic acid derived from cancer cells. In this case, the cancer cell marker composition may be used as a vaccine composition and may further include an adjuvant (component) to boost the expected effect. The specific limitations regarding the cancer, vaccine, or adjuvant overlap with those described in the "immunity-boosting", and will be omitted below to avoid excessive complexity of the present specification.

As used herein, the term "kit" is a concept of a set including all or part of a preparation for detecting a target substance. The kit may be applied to a biological sample isolated from a subject. The biological sample isolated from the subject may be one or more selected from the group consisting of blood, saliva, tissues, cells, sputum, bronchoalveolar lavage fluid, and urine. The biological sample may be a dried type of liquid or tissue sample, or a sample contained in a liquid. In the present invention, the purpose of the kit may be to confirm the presence or absence of a specific gene or nucleic acid from a biological sample, and the kit may preferably include a sequence having 70% or more homology with any one selected from the group consisting of SEQ ID NO: 1 to SEQ ID NO: 18, and SEQ ID NO: 20 to SEQ ID NO: 37 of the present invention. For example, as a kit for detecting an A gene or nucleic acid in a biological sample isolated from a subject, a kit including a B nucleic acid complementarily binding to the A gene or nucleic acid as all or part of the preparation may include a sequence having 70% or more homology with any one selected from the group consisting of SEQ ID NO: 1 to SEQ ID NO: 18, and SEQ ID NO: 20 to SEQ ID NO: 37 of the present invention, together with the B nucleic acid, for stabilizing the B nucleic acid.

As used herein, the term "pharmaceutical composition" refers to a concept of a composition administered for the prevention or treatment of a desired disease. The disease may be selected without limitation as long as requiring prevention or treatment, but may be prevented or treated by regulating gene expression for the purpose of the present invention. Here, the gene may be a gene that directly affects the prevention or treatment of a disease, such as a hereditary disease, or may be a gene that can be expected to have an indirect effect of preventing or treating a disease through immunity boosting *in vivo,* such as immune cell stimulation. In the present invention, the pharmaceutical composition may include a sequence having 70% or more homology with any one selected from the group consisting of SEQ ID NO: 1 to SEQ ID NO: 18, and SEQ ID NO: 20 to SEQ ID NO: 37 of the present invention for regulating the expression of a desired gene. For example, a pharmaceutical composition for the prevention or treatment of cancer may be a pharmaceutical composition including a sequence having at least 70% homology with any one selected from the group consisting of SEQ ID NO: 1 to SEQ ID NO: 18, and SEQ ID NO: 20 to SEQ ID NO: 37 and a nucleic acid for inhibiting the expression of an oncogene. Alternatively, the pharmaceutical composition may be a pharmaceutical composition including a sequence having at least 70% homology with any one selected from the group consisting of SEQ ID NO: 1 to SEQ ID NO: 18, and SEQ ID NO: 20 to SEQ ID NO: 37 and a nucleic acid for enhancing the expression of an antioncogene. Alternatively, the pharmaceutical composition may be a pharmaceutical composition including a sequence having at least 70% homology with any one selected from the group consisting of SEQ ID NO: 1 to SEQ ID NO: 18, and SEQ ID NO: 20 to SEQ ID NO: 37 and a nucleic acid for stimulating the immune cell. Alternatively, the pharmaceutical composition may be a pharmaceutical composition including a sequence having at least 70% homology with any one selected from the group consisting of SEQ ID NO: 1 to SEQ ID NO: 18, and SEQ ID NO: 20 to SEQ ID NO: 37 and a nucleic acid for a cancer cell marker. Alternatively, the pharmaceutical composition may be a pharmaceutical composition including a sequence having at least 70% homology with any one selected from the group consisting of SEQ ID NO: 1 to SEQ ID NO: 18, and SEQ ID NO: 20 to SEQ ID NO: 37 and a patient-specific neoantigen. Alternatively, the pharmaceutical composition may be a pharmaceutical composition of the synthetic long peptide (SLP) including a sequence having at least 70% homology with any one selected from the group consisting of SEQ ID NO: 1 to SEQ ID NO: 18, and SEQ ID NO: 20 to SEQ ID NO: 37 and a plurality of patient-specific neoantigens. The pharmaceutical composition may be in the form of capsules, tablets, granules, injections, ointments, powders or beverages, and may target humans. The pharmaceutical composition of the present invention may be formulated and used in the form of oral formulations such as powders, granules, capsules, tablets, and aqueous suspensions, external preparations, suppositories, and injections, according to conventional methods, but preferably suppositories or injections. In addition, the pharmaceutical composition of the present invention may include a pharmaceutically acceptable carrier. The pharmaceutically acceptable carrier may be used with a binder, a lubricant, a disintegrant, an excipient, a solubilizer, a dispersant, a stabilizer, a suspending agent, a pigment, a flavoring, and the like during oral administration, may be mixed and used with a buffering agent, a preservative, a painless agent, a solubilizer, an isotonic agent, a stabilizer, and the like in the case of injections, and may be used with a base, an excipient, a lubricant, a preservative, and the like in the case of topical administration. The formulations of the pharmaceutical composition of the present invention may be prepared variously in combination with the pharmaceutically acceptable carriers described above. For example, for oral administration, the pharmaceutical composition may be formulated in the form of tablets, troches, capsules, elixirs, suspensions, syrups, wafers, etc., and for injections, the pharmaceutical composition may be formulated in the form of a single dose ampoule or a multiple dose form. In addition, the pharmaceutical composition may be formulated into solutions, suspensions, tablets, capsules, sustained release agents, and the like. Meanwhile, examples of the carrier, the excipient, and the diluent suitable for the formulations may be used with lactose, dextrose, sucrose, sorbitol, mannitol, xylitol, erythritol, maltitol, starch, acacia gum, alginate, gelatin, calcium phosphate, calcium silicate, cellulose, methylcellulose, microcrystalline cellulose, polyvinylpyrrolidone, water, methylhydroxybenzoate, propylhydroxybenzoate, talc, magnesium stearate, mineral oils, or the like. In addition, the pharmaceutical composition may further include fillers, anti-coagulating agents, lubricants, wetting agents, flavorings, emulsifiers, preservatives, and the like. The pharmaceutical composition according to the present invention may be administered via oral, intravenous, intramuscular, intraarterial, intramedullary, intrathecal, intracardiac, transdermal, subcutaneous, intraperitoneal, intranasal, enteral, topical, sublingual or rectal route, but preferably parenteral administration as described above. The parenteral administration includes subcutaneous, intradermal, intravenous, intramuscular, intraarticular, intrasynovial, intrasternal, intrathecal, intralesional and intracranial injection or infusion techniques. The pharmaceutical composition of the present invention may also be administered in the form of a suppository for rectal administration. The pharmaceutical composition of the present invention may be variously changed according to various factors, including the activity of a specific compound used, age, body weight, general health, sex, diet, administration time, administration route, excretion rate, drug formulation, and the severity of a specific disease to be prevented or treated. The dose of the pharmaceutical composition varies depending on the condition and body weight of a patient, the degree of a disease, a drug form, and administration route and period, but may be appropriately selected by those skilled in the art. The dose may be administered once a day or several times a day. The dose does not limit the scope of the present invention in any aspect.

In an embodiment of the present invention, there is provided an artificial nucleic acid including a sequence having at least 70% homology with any one selected from the group consisting of SEQ ID NO: 1 to SEQ ID NO: 18, and SEQ ID NO: 20 to SEQ ID NO: 37. The sequence may be an untranslated region sequence. The sequence may be a sequence having at least 90% homology with any one selected from the group consisting of SEQ ID NO: 1 to SEQ ID NO: 18, and SEQ ID NO: 20 to SEQ ID NO: 37. The sequence may be a sequence having at least 95% homology with any one selected from the group consisting of SEQ ID NO: 1 to SEQ ID NO: 18, and SEQ ID NO: 20 to SEQ ID NO: 37. The artificial nucleic acid may include a sequence having at least 70% homology with any one selected from the group consisting of SEQ ID NO: 1 to SEQ ID NO: 18; and a sequence having at least 70% homology with any one selected from the group consisting of SEQ ID NO: 20 to SEQ ID NO: 37. The artificial nucleic acid may include a sequence having at least 70% homology with any one selected from the group consisting of SEQ ID NO: 1 to SEQ ID NO: 18 and located at 5'; and a sequence having at least 70% homology with any one selected from the group consisting of SEQ ID NO: 20 to SEQ ID NO: 37 and located at 3'. In addition, the artificial nucleic acid may further include at least one of an open reading frame sequence, a promoter sequence, a Kozak sequence, and a poly A tail sequence.

In another embodiment of the present invention, there is provided a vector including the artificial nucleic acid, in which the vector is a plasmid vector or a viral vector.

In yet another embodiment of the present invention, there is provided a transfectant transfected with the vector, in which the transfectant is a virus, a bacterium, a plant cell, or an animal cell.

In yet another embodiment of the present invention, there is provided a composition for stabilizing nucleic acids including the artificial nucleic acid.

In yet another embodiment of the present invention, there is provided an immunity-boosting composition including the artificial nucleic acid, in which the immunity-boosting composition is a vaccine composition.

In yet another embodiment of the present invention, there is provided a cancer cell marker composition including the artificial nucleic acid, in which the cancer cell marker composition is a vaccine composition.

In yet another embodiment of the present invention, there is provided a kit for detecting nucleic acids including the artificial nucleic acid.

In yet another embodiment of the present invention, there is provided a pharmaceutical composition for preventing or treating a disease including the artificial nucleic acid.

In yet another embodiment of the present invention, there is provided a method for selecting an untranslated region sequence for stabilizing nucleic acids, including (a) selecting genes having the top 20% of expression levels from a database; (b) primary selecting untranslated region sequences of the selected genes; and (c) secondary selecting an untranslated region sequence having no secondary structure from the selected untranslated region sequences. In step (c), a method for selecting the untranslated region sequence having no secondary structure may be performed by calculating a minimum free energy (MFE) and selecting a 5' UTR sequence having a value greater than -10.

In yet another embodiment of the present invention, there is provided a method for selecting an untranslated region sequence for stabilizing nucleic acids, including (a) selecting genes having the top 20% of expression levels from a database; (b) primary selecting untranslated region sequences of the selected genes; and (c) secondary selecting an untranslated region sequence having no AU rich element (ARE) from the selected untranslated region sequences. The method for selecting the untranslated region sequence for stabilizing nucleic acids may further include, after step (b), secondary selecting an untranslated region sequence of the shortest sequence if there are a plurality of isoforms of the same gene among the selected untranslated region sequences; and/or clustering miRNAs using binding energy between the selected UTR sequence and miRNA, and then further selecting an untranslated region sequence based on the information.

In yet another embodiment of the present invention, there is provided a method for manufacturing an artificial nucleic acid, including: manufacturing an artificial nucleic acid so as to include a sequence having at least 70% homology with an untranslated region sequence selected by at least one of the methods for selecting the untranslated region sequence for stabilizing nucleic acids.

Hereinafter, the present invention will be described in detail by Examples.

### [Effects of the Invention]

According to the present invention, the UTR sequence has excellent effects of stabilizing nucleic acids and promoting the expression of a target protein, and thus is expected to be greatly used in the health/medical field.

### [Brief Description of Drawings]

FIG. 1 is a diagram illustrating a plasmid DNA construct structure for IVT mRNA synthesis with a selection gene UTR according to an embodiment of the present invention.
FIG. 2 is a schematic diagram of a plasmid vector using a selection gene according to an embodiment of the present invention. Representatively, an example in which an RGS5 gene is used is illustrated, but plasmid vectors of other genes also have the same structure with only changed genes.
FIG. 3 illustrates results of confirming linearization of plasmid DNA vectors of a UTR candidate group before *in vitro* transcription (IVT) according to an embodiment of the present invention.
FIG. 4 illustrates results of analyzing d2eGFP fluorescence expression according to selection gene UTR sequences using fluorescence imaging according to an embodiment of the present invention.
FIG. 5 illustrates results of analyzing d2eGFP fluorescence expression according to selection gene UTR sequences using flow cytometry according to an embodiment of the present invention.
FIG. 6 illustrates results of confirming transfection and translation efficiencies according to selection gene UTR sequences using flow cytometry according to an embodiment of the present invention.

### [Modes of the Invention]

Hereinafter, the present invention will be described in more detail with reference to Examples. These Examples are to explain the present invention in more detail, and it will be apparent to those skilled in the art that the scope of the present invention is not limited by these Examples in accordance with the gist of the present invention.

### Example 1. Design and selection of UTR candidate sequences

In order to select UTR sequences that increased the intracellular stability and translation efficiency of mRNA vaccines, 1,689 genes with high expression levels were selected based on the genotype-tissue expression (GTEx), a database of gene expression information in normal tissues, using an *in silico* method, assuming that genes with high expression levels *in vivo* would include UTRs that increased the stability of *in vivo* mRNA and had high translation efficiency. The 1,689 genes corresponded to the top 10% of 16,903 genes in the database. Thereafter, candidate UTR sequences for stabilizing nucleic acids were selected from 5' UTR and 3' UTR sequences of the selected genes.

Specifically, for the 5' UTR, a relatively short sequence of 20 nt to 60 nt was selected. If the length of the 5' UTR is long, difficulties in vaccine synthesis may occur and undesirable structural biological reactions may occur. Since an antigen different from the design may be synthesized when an upstream AUG is present in the selected sequence, cases where an upstream AUG is present were excluded. In addition, in order to additionally remove 5' UTRs with structural characteristics that inhibit translation (formation of secondary structures in 5' UTRs), a minimum free energy (MFE) was calculated and 5' UTR sequences with MFE values greater than - 10 were selected. As a result, a total of 360 5' UTR candidate sequences were selected. In the case of 3' UTR, 3' UTRs satisfying 20 nt to 300 nt in length were first selected for efficient *in vitro* transcription (IVT), and if many isoforms of the same gene existed, 1,077 3' UTR candidate sequences were first selected by selecting the shortest 3' UTR among the isoforms. In order to identify a binding pattern with miRNA that affected the stability of mRNA for the candidate sequences, the binding energy between 1,077 candidate 3' UTR sequences and 2,657 miRNAs was calculated (IntaRNA 2.0), and clustering was performed using binding miRNA types and its energy with a cola R package. Here, UTR sequences classified into the same cluster as UTR sequences known through various prior literatures in the art were secondary selected, and then genes containing AU rich elements (AREs) involved in mRNA degradation in 3' UTRs were excluded by referring to the AU-rich element database (ARED-Plus). As a result, a total of 217 3' UTR candidate sequences were selected.

### Example 2. Optimization of selected UTR candidates

Among the 360 5' UTR and 217 3' UTR sequences selected in Example 1, 40 5' UTRs and 3' UTRs derived from the same gene were selected. Among the UTRs, 9 5' UTRs and 3' UTRs were reselected considering the expression level of the gene, and sequence optimization was performed to have a strong Kozak sequence. As a result, the selected genes were CLPS (colipase), APOC3 (apolipoprotein C-III), REG3A (regenerating family member 3 alpha), TNNC1 (troponin C type 1), HPX (hemopexin), RGS5 (regulator of G protein signaling 5), IFI30 (IFI30 lysosomal thiol reductase), CYP11A1 (cytochrome P450 family 11 subfamily A member 1), and CYC1 (cytochrome C1). Among the UTRs of the genes, the UTR sequences for which the Kozak sequences were optimized were shown in Table 1 (5' UTR) and Table 2 (3' UTR) below. As a control UTR for these, conventional UTR sequences known in the art were used.

**[Table 1]**

| SEQ ID NO: | Gene | Nucleic acid type | Sequence |
|---|---|---|---|
| SEQ ID NO: 1 | REG3A | DNA | AAACCATACCATATCCCACCAGAGAGT CGCAGCCACC |
| SEQ ID NO: 2 | REG3A | RNA | AAACCAUACCAUAUCCCACCAGAGAG UCGCAGCCACC |
| SEQ ID NO: 3 | HPX | DNA | GTCCTGTGGCCTCTGCAGGCCACC |
| SEQ ID NO: 4 | HPX | RNA | GUCCUGUGGCCUCUGCAGGCCACC |
| SEQ ID NO: 5 | RGS5 | DNA | AGAAGTGTCTGACAAAACAAAGAAACA AAACCTCAGGCCACC |
| SEQ ID NO: 6 | RGS5 | RNA | AGAAGUGUCUGACAAAACAAAGAAAC AAAACCUCAGGCCACC |
| SEQ ID NO: 7 | CLPS | DNA | GTCTCCCGCCACCCACACCAGCTGTCCC AGCCACC |
| SEQ ID NO: 8 | CLPS | RNA | GUCUCCCGCCACCCACACCAGCUGUCC |
| | | | CAGCCACC |
| SEQ ID NO: 9 | APOC3 | DNA | CTGCTCAGTTCATCCCTAGAGGCAGCTG CTCCAGGAACAGAGCCACC |
| SEQ ID NO: 10 | APOC3 | RNA | CUGCUCAGUUCAUCCCUAGAGGCAGC UGCUCCAGGAACAGAGCCACC |
| SEQ ID NO: 11 | TNNC1 | DNA | AGCAAGCTGTCCTGTGAGCCGCCACC |
| SEQ ID NO: 12 | TNNC1 | RNA | AGCAAGCUGUCCUGUGAGCCGCCACC |
| SEQ ID NO: 13 | IFI30 | DNA | GCAGTCGCCACACCTTTGCCCCTGCCAC C |
| SEQ ID NO: 14 | IFI30 | RNA | GCAGUCGCCACACCUUUGCCCCUGCCA CC |
| SEQ ID NO: 15 | CYP11 A1 | DNA | CCGCTGAAGTGGAGCAGGTACAGTCAC AGCTGTGGGGCCACC |
| SEQ ID NO: 16 | CYP11 A1 | RNA | CCGCUGAAGUGGAGCAGGUACAGUCA CAGCUGUGGGCCACC |
| SEQ ID NO: 17 | CYC1 | DNA | GGCGGCCGCGCGGAGGAGGCCACC |
| SEQ ID NO: 18 | CYC1 | RNA | GGCGGCCGCGCGGAGGAGGCCACC |
| SEQ ID NO: 19 | Positive control | DNA | GAATAAACTAGTATTCTTCTGGTCCCCA CAGACTCAGAGAGAACCCGCCACC |

**[Table 2]**

| SEQ ID NO: | Gene | Nucleic acid type | Sequence |
|---|---|---|---|
| SEQ ID NO: 20 | REG3A | DNA | TGCAGGAGGGAAGTCAGCAGCCTGTGT TTGGTGTGCAACTCATCATGGGCATGA GACCAGTGTGAGGACTCACCCTGGAAG AGAATATTCGCTTAATTCCCCCAACCTG ACCACCTCATTCTTATCTTTCTTCTGTTT CTTCCTCCCCGCTGTCATTTCAGTCTCTT CATTTTGTCATACGGCCTAAGGCTTTAA AGAGCAATAAAATTTTTAGTCTGC |
| SEQ ID NO: 21 | REG3A | RNA | UGCAGGAGGGAAGUCAGCAGCCUGUG UUUGGUGUGCAACUCAUCAUGGGCAU GAGACCAGUGUGAGGACUCACCCUGG AAGAGAAUAUUCGCUUAAUUCCCCCA ACCUGACCACCUCAUUCUUAUCUUUCU UCUGUUUCUUCCUCCCCGCUGUCAUUU CAGUCUCUUCAUUUUGUCAUACGGCC UAAGGCUUUAAAGAGCAAUAAAAUUU UUAGUCUGC |
| SEQ ID NO: 22 | HPX | DNA | GGGGCCTTCTGACATGAGTCTGGCCTG GCCCCACCTCCTAGTTCCTCATAATAAA GACAGATTGCTTCTTCGCTTCTCACTGA GGGGCCTTCTGACATGAGTCTGGCCTG GCCCCACCTCCCCAGTTTCTCATAATAA AGACAGATTGCTTCTTCACTTGAA |
| SEQ ID NO: 23 | HPX | RNA | GGGGCCUUCUGACAUGAGUCUGGCCU GGCCCCACCUCCUAGUUCCUCAUAAUA AAGACAGAUUGCUUCUUCGCUUCUCA CUGAGGGGCCUUCUGACAUGAGUCUG GCCUGGCCCCACCUCCCCAGUUUCUCA UAAUAAAGACAGAUUGCUUCUUCACU UGAA |
| SEQ ID NO: 24 | RGS5 | DNA | TAATTTAGCCAGGCTATGAAATCATCCT GTGAGTTATTTCCTCCATAATAACCCTG CATTTCCCATTAATCTACATATCTTCCC ACAGCAGCTTTGCTCAGTGATACCCAC ATGGGAAAAATCCCAGGGGATGTTGCT TACTCTTTTTGCCCACACTGCTTTGGAT ACTTATCTACTGTCCGAAGGCCTTCTTT CCCCACTCAATTCTTCCTGCCCTGTTAT TAA |
| SEQ ID NO: 25 | RGS5 | RNA | UAAUUUAGCCAGGCUAUGAAAUCAUC CUGUGAGUUAUUUCCUCCAUAAUAAC CCUGCAUUUCCCAUUAAUCUACAUAU CUUCCCACAGCAGCUUUGCUCAGUGA |
| | | | UACCCACAUGGGAAAAAUCCCAGGGG AUGUUGCUUACUCUUUUUGCCCACAC UGCUUUGGAUACUUAUCUACUGUCCG AAGGCCUUCUUUCCCCACUCAAUUCUU CCUGCCCUGUUAUUAA |
| SEQ ID NO: 26 | CLPS | DNA | GACTGCCCACCCACTCCCACACCTAGC CCAGAATGCTGTAGGCCACTAGGCGCA GGGGCATCTCTCCCCTGCTCCAGCGCAT CTCCCGGGCTGGCCACCTCCTTGACCAG CATATCTGTTTTCTGATTGCGCTCTTCA CAATTAAAGGCCTCCTGCAAACCTTT |
| SEQ ID NO: 27 | CLPS | RNA | GACUGCCCACCCACUCCCACACCUAGC CCAGAAUGCUGUAGGCCACUAGGCGC AGGGGCAUCUCUCCCCUGCUCCAGCGC AUCUCCCGGGCUGGCCACCUCCUUGAC CAGCAUAUCUGUUUUCUGAUUGCGCU CUUCACAAUUAAAGGCCUCCUGCAAA CCUUU |
| SEQ ID NO: 28 | APOC3 | DNA | GACCTCAATACCCCAAGTCCACCTGCCT ATCCATCCTGCGAGCTCCTTGGGTCCTG CAATCTCCAGGGCTGCCCCTGTAGGTTG CTTAAAAGGGACAGTATTCTCAGTGCT CTCCTACCCCACCTCATGCCTGGCCCCC CTCCAGGCATGCTGGCCTCCCAATAAA GCTGGACAAGAAGCTGCTATGA |
| **SEQ** ID NO: 29 | APOC3 | RNA | GACCUCAAUACCCCAAGUCCACCUGCC UAUCCAUCCUGCGAGCUCCUUGGGUCC UGCAAUCUCCAGGGCUGCCCCUGUAG GUUGCUUAAAAGGGACAGUAUUCUCA GUGCUCUCCUACCCCACCUCAUGCCUG GCCCCCCUCCAGGCAUGCUGGCCUCCC AAUAAAGCUGGACAAGAAGCUGCUAU GA |
| SEQ ID NO: 30 | TNNC1 | DNA | ATGCTGACCTTCACCCAGAGCTGCCTAT GCCCAGCCTCCAACTCCAGCTGAGTCCT |
| | | | GGGGTTGGGGAGGGGGTCGGGGTCCCA GGACCTGAGCCTGGCCATGTCCTCAAC CCCAAATCCCCCGACTCCCTCCCCAGAT CTGTCCTGGGGGATGCAAATAAAGCCT GCTCTCCCAA |
| SEQ ID NO: 31 | TNNC1 | RNA | AUGCUGACCUUCACCCAGAGCUGCCUA UGCCCAGCCUCCAACUCCAGCUGAGUC CUGGGGUUGGGGAGGGGGUCGGGGUC CCAGGACCUGAGCCUGGCCAUGUCCUC AACCCCAAAUCCCCCGACUCCCUCCCC AGAUCUGUCCUGGGGGAUGCAAAUAA AGCCUGCUCUCCCAA |
| SEQ ID NO: 32 | IFI30 | DNA | TGGCCGGTGAGCTGCGGAGAGCTCATG GAAGGCGAGTGGGAACCCGGCTGCCTG CCTTTTTTTCTGATCCAGACCCTCGGCA CCTGCTACTTACCAACTGGAAAATTTTA TGCATCCCATGAAGCCCAGATACACAA AATTCCACCCCATGATCAAGAATCCTG CTCCACTAAGAATGGTGCTAAAGTAAA ACTAGTTTAATAAGC |
| SEQ ID NO: 33 | IFI30 | RNA | UGGCCGGUGAGCUGCGGAGAGCUCAU GGAAGGCGAGUGGGAACCCGGCUGCC UGCCUUUUUUUCUGAUCCAGACCCUC GGCACCUGCUACUUACCAACUGGAAA AUUUUAUGCAUCCCAUGAAGCCCAGA UACACAAAAUUCCACCCCAUGAUCAA GAAUCCUGCUCCACUAAGAAUGGUGC UAAAGUAAAACUAGUUUAAUAAGC |
| SEQ ID NO: 34 | CYP11 A1 | DNA | TCAGAGAGGATGGCCTGCAGCCACATG GGAGGAAGGCCCAGGGGTGGGGCCCAT GGGGTCTCTGCATCTTCAGTCGTCTGTC CCAAGTCCTGCTCCTTTCTGCCCAGCCT GCTCAGCAGGTTGAATGGGTTCTCAGT GGTCACCTTCCTCAGCTCAGCTGGGCCA CTCCTCTTCACCCACCCCATGGAGACAA |
| | | | TAAACAGCTGAACCATG |
| SEQ ID NO: 35 | CYP11 A1 | RNA | UCAGAGAGGAUGGCCUGCAGCCACAU GGGAGGAAGGCCCAGGGGUGGGGCCC AUGGGGUCUCUGCAUCUUCAGUCGUC UGUCCCAAGUCCUGCUCCUUUCUGCCC AGCCUGCUCAGCAGGUUGAAUGGGUU CUCAGUGGUCACCUUCCUCAGCUCAGC UGGGCCACUCCUCUUCACCCACCCCAU GGAGACAAUAAACAGCUGAACCAUG |
| SEQ ID NO: 36 | CYC1 | DNA | CCCTGTCCAGTGTCTGCTTGCCATCCTG CCAGAACAGGCCCTCAAGCCCAAGAGC CATCCCAGGCCTGTTCAGGCCTCAGCTA AGCCTCTCTTCATCTGGAAGAAGAGGC AAGGGGGCAGGAGACCAGGCTCTAGCT CTGGGCCCTCCTTCAGCCCCCATCATGG GAATAAATTAATTTTCTCAATGTA |
| SEQ ID NO: 37 | CYC1 | RNA | CCCUGUCCAGUGUCUGCUUGCCAUCCU GCCAGAACAGGCCCUCAAGCCCAAGAG CCAUCCCAGGCCUGUUCAGGCCUCAGC UAAGCCUCUCUUCAUCUGGAAGAAGA GGCAAGGGGGCAGGAGACCAGGCUCU AGCUCUGGGCCCUCCUUCAGCCCCCAU CAUGGGAAUAAAUUAAUUUUCUCAAU GUA |
| SEQ ID NO: 38 | Positive control | DNA | CTCGAGCTGGTACTGCATGCACGCAAT GCTAGCTGCCCCTTTCCCGTCCTGGGTA CCCCGAGTCTCCCCCGACCTCGGGTCCC AGGTATGCTCCCACCTCCACCTGCCCCA CTCACCACCTCTGCTCGTTCCAGACACC TCCCAAGCACGCAGCAATGCAGCTCAA AACGCTTAGCCTAGCCACACCCCCACG GGAAACAGCAGTGATTAACCTTTAGCA ATAAACGAAAGTTTAACTAAGCTATAC TAACCCCAGGGTTGGTCAATTTCGTGCC AGCCACACCCTGGAGCTAGCAA |

In Tables 1 and 2 above, if the sequence is RNA, U (uracil) in the sequence may be represented by T in the sequence list.

### Example 3. Performance evaluation of selected UTR candidates

### Example 3-1. Preparation of plasmid DNA for IVT mRNA synthesis with UTR

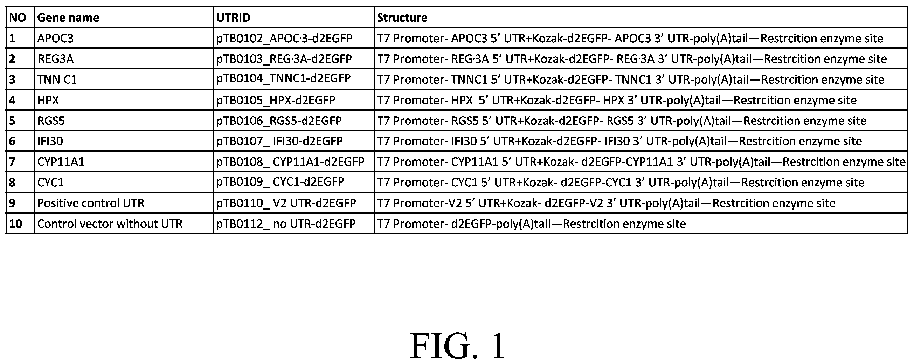

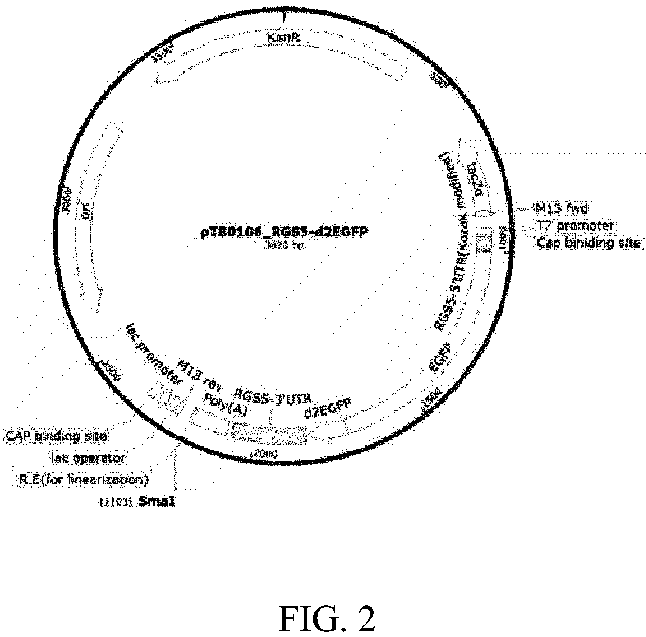

In order to evaluate the performance of the UTR optimized candidate sequences selected in Example 2 above, IVT template DNA was prepared to produce IVT mRNA through RNA polymerase (T7 polymerase) reaction and then transfect the IVT mRNA into K562 cell lines to compare and analyze the intracellular mRNA stability and protein expression ability. Specifically, plasmid DNA for producing IVT mRNA in the form of each UTR candidate sequence inserted was prepared, and a vector for producing IVT mRNA was designed and prepared based on pUC57-kan IVT plasmid vector backbone DNA, considering a linearization method and a Cap binding site. A DNA construct was prepared using an In-fusion cloning technique, and prepared with primers in the form of extended 3' and 5' terminal sequences (15 bp) of a linkage site so as to be shared during insert fragment amplification. After PCR amplification, the terminal regions were ligated and cloned using a linearized plasmid vector and an In-fusion cloning kit. Since the reaction-induced plasmid DNA contained a kanamycin resistant gene, the vector was designed by performing single colony selection on an antibiotic medium containing kanamycin, and inserting d2EGFP, which had a short half-life, into a coding sequence (CDS) region to facilitate comparison of protein expression between candidates. Thereafter, based on the prepared IVT vector, DNA was prepared in the form of each selected gene type inserted to produce the final version, an IVT template. The prepared UTR candidate constructs have the same sequence except for the UTR sequence. The structure of the plasmid DNA construct for IVT mRNA synthesis containing the prepared UTR was shown in FIGS. 1 and 2. FIG. 2 representatively illustrates a schematic diagram of a plasmid vector using an RGS5 gene, but plasmid vectors of other genes also have the same structure with only changed genes.

### Example 3-2. Plasmid DNA linearization and confirmation

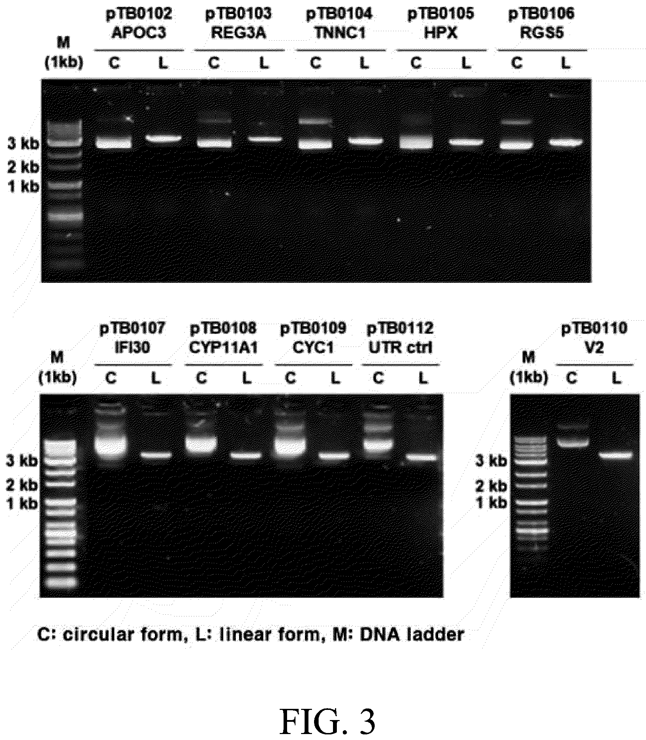

Control plasmid DNA and 8 kinds of plasmid DNA circular forms containing selected gene UTR candidates were linearized. Plasmid DNA linearization was performed using a restriction enzyme, and for this end, Sma I restriction enzyme (Enzynomics, cat# R015S) was used. The linearized plasmid DNA cut by the restriction enzyme Sma I was confirmed to change into a linearized form through agarose gel electrophoresis. As a result of the experiment, it was confirmed that all plasmid DNAs containing the selected gene UTR sequence were changed from a circular form to a linear form by the Sma I restriction enzyme. The results were shown in FIG. 3.

### Example 3-3. mRNA production and performance evaluation using linearized plasmid DNA

### (1) In vitro transcription

IVT mRNA synthesis was performed using mMESSAGE mMACHINE^{®} T7 transcription kit (Invitrogen, cat# AM1344) with 8 types of linear plasmid DNAs containing selected gene UTR sequences. IVT was performed by RNA polymerase T7 polymerase to synthesize mRNA, and the 8 types of linear plasmid DNAs contained a T7 promoter sequence. Nucleoside triphosphates (NTPs) and Cap analog were added during the IVT process, and a 5' cap was attached to IVT mRNA 5' end using a co-transcriptional capping method. The concentration of the finally synthesized IVT mRNA was measured using a nanodrop device, and purification was performed using MEGAclear^{™} transcription clean-up kit (Invitrogen, cat# AM1908).

### (2) IVT mRNA transfection

The translation efficiency was evaluated by transfecting the UTR candidate IVT mRNA encoding a marker reporter gene, d2EGFP sequence into a human myeloid leukemia cell line (K-562; human lymphoblast). The UTR candidate IVT mRNA transfection was performed using Lipofectamine MessengerMax (Invitrogen, cat# LMRNA015).

### (3) Verification of translational efficiency using d2EGFP-encoding IVT mRNA

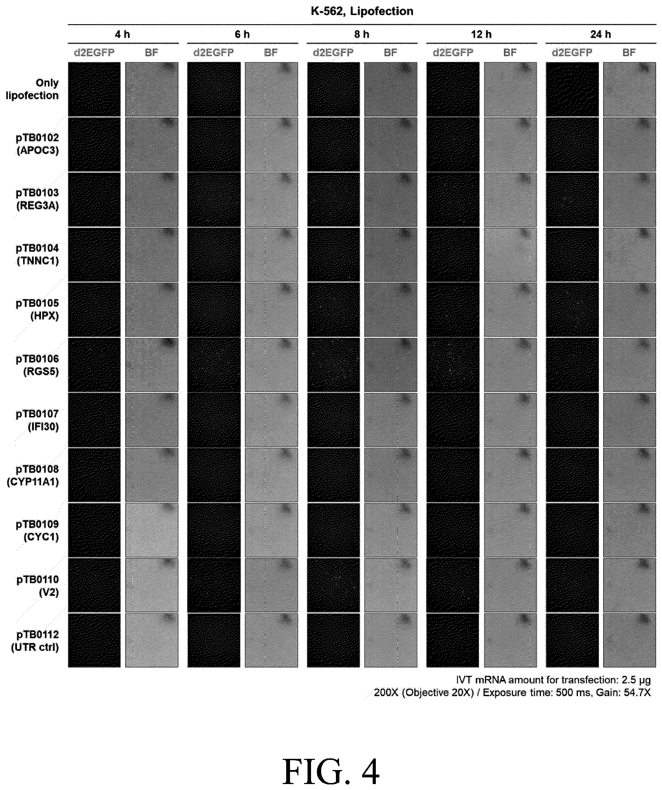

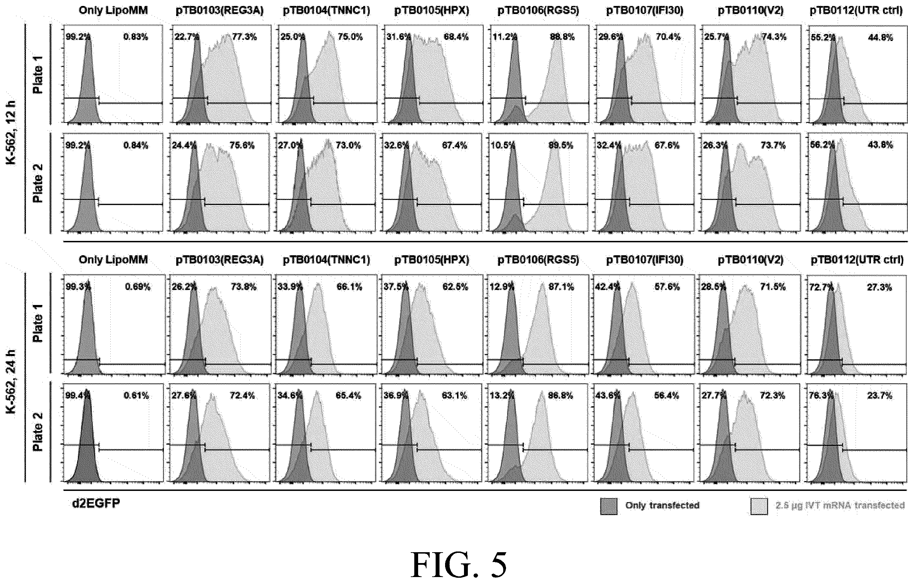

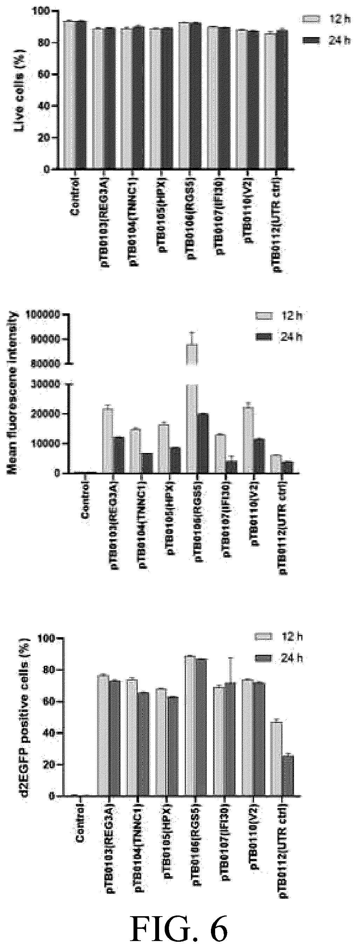

The IVT mRNA synthesized from 8 types of linear plasmid DNAs containing the control UTR and the selected gene UTR sequences contained the marker reporter gene d2EGFP (see FIG. 1). After transfection into the human myeloid leukemia cell line, it was confirmed that d2EGFP was expressed by IVT mRNA translation. Therefore, 2.5 µg of 8 types of d2EGFP-encoding IVT mRNAs containing the selected gene UTR sequences were transfected into 5 × 10⁵ cells of the human myeloid leukemia cell line using Lipofectamine MessengerMax. Since the translation efficiency of d2EGFP-containing IVT mRNA varied depending on the selected gene UTR sequence, it is expected that there will be differences even in the expression level of d2EGFP. Therefore, the changes in d2EGFP expression by time were analyzed by fluorescence imaging and flow cytometry at time points of 4, 6, 8, 12, and 24 hours after transfection. The results were illustrated in FIGS. 4 and 5. In the case of the fluorescence imaging analysis, since d2EGFP expression was not found in a negative control group treated only with Lipofectamine MM at 12 hours after transfection in the previous study, fluorescence microscope conditions (Exposure time, Gain) were determined through this background confirmation. In addition, as a result of confirming d2EGFP expression using a positive (V2 UTR (pTB0110)) control confirmed in the previous study, d2EGFP expression was barely observed in no UTR-control (pTB0112) where the UTR sequence was removed, which indicated that the experiment was performed well. As the experiment result, UTRs of APOC3, REG3A, TNNC1, HPX, and RGS5 genes showed higher d2EGFP expression than a positive control V2 UTR (pTB0110). In particular, it was confirmed that the UTR expression of REG3A, HPX, and RGS5 genes was very remarkable, and in the case of UTRs of the REG3A, HPX, and RGS5 genes, d2EGFP expression was stably maintained for up to 24 hours. In the case of the flow cytometry analysis, only live cells (%) were selected from total cells, and only K-562 expressing d2EGFP was classified among the cells and a difference in d2EGFP expression according to the selected gene UTR sequences was compared and analyzed. As a result, it was confirmed that d2EGFP-encoding IVT mRNA transfection through lipofection maintained high cell viability for up to 24 hours in all UTR sequences. The d2EGFP expression was highest in an RGS5 UTR group, but there was no significant difference in all d2EGFP positive cells by time. The results were shown in FIG. 6.

As described above, specific parts of the present invention have been described in detail, and it will be apparent to those skilled in the art that these specific techniques are merely preferred embodiments, and the scope of the present invention is not limited thereto. Therefore, the substantial scope of the present invention will be defined by the appended claims and their equivalents.

## Claims

1. An artificial nucleic acid comprising a sequence having at least 70% homology with any one selected from the group consisting of SEQ ID NO: 1 to SEQ ID NO: 18, and SEQ ID NO: 20 to SEQ ID NO: 37.

2. The artificial nucleic acid of claim 1, wherein the sequence is an untranslated region sequence.

3. The artificial nucleic acid of claim 1, wherein the sequence is a sequence having at least 90% homology with any one selected from the group consisting of SEQ ID NO: 1 to SEQ ID NO: 18, and SEQ ID NO: 20 to SEQ ID NO: 37.

4. The artificial nucleic acid of claim 1, wherein the sequence is a sequence having at least 95% homology with any one selected from the group consisting of SEQ ID NO: 1 to SEQ ID NO: 18, and SEQ ID NO: 20 to SEQ ID NO: 37.

5. The artificial nucleic acid of claim 1, wherein the artificial nucleic acid includes a sequence having at least 70% homology with any one selected from the group consisting of SEQ ID NO: 1 to SEQ ID NO: 18; and
a sequence having at least 70% homology with any one selected from the group consisting of SEQ ID NO: 20 to SEQ ID NO: 37.

6. The artificial nucleic acid of claim 5, wherein the artificial nucleic acid includes a sequence having at least 70% homology with any one selected from the group consisting of SEQ ID NO: 1 to SEQ ID NO: 18 and located at 5'; and
a sequence having at least 70% homology with any one selected from the group consisting of SEQ ID NO: 20 to SEQ ID NO: 37 and located at 3'.

7. The artificial nucleic acid of claim 1, further comprising:
at least one of an open reading frame sequence, a promoter sequence, a Kozak sequence, and a poly A tail sequence.

8. A vector comprising the artificial nucleic acid of claim 1.

9. The vector of claim 8, wherein the vector is a plasmid vector or a viral vector.

10. A transfectant transfected with the vector of claim 8.

11. The transfectant of claim 10, wherein the transfectant is a virus, a bacterium, a plant cell, or an animal cell.

12. A composition for stabilizing nucleic acids comprising the artificial nucleic acid of claim 1.

13. An immunity-boosting composition comprising the artificial nucleic acid of claim 1.

14. The immunity-boosting composition of claim 13, wherein the immunity-boosting composition is a vaccine composition.

15. A cancer cell marker composition comprising the artificial nucleic acid of claim 1.

16. The cancer cell marker composition of claim 15, wherein the cancer cell marker composition is a vaccine composition.

17. A kit for detecting nucleic acids comprising the artificial nucleic acid of claim 1.

18. A pharmaceutical composition for preventing or treating a disease comprising the artificial nucleic acid of claim 1.

19. A method for selecting an untranslated region sequence for stabilizing nucleic acids comprising:
(a) selecting genes having the top 20% of expression levels from a database;
(b) primary selecting untranslated region sequences of the selected genes; and
(c) secondary selecting an untranslated region sequence having no secondary structure from the selected untranslated region sequences.

20. A method for selecting an untranslated region sequence for stabilizing nucleic acids, comprising:
(a) selecting genes having the top 20% of expression levels from a database;
(b) primary selecting untranslated region sequences of the selected genes; and
(c) secondary selecting an untranslated region sequence having no AU rich element (ARE) from the selected untranslated region sequences.

21. A method for manufacturing an artificial nucleic acid comprising:
(a) selecting the untranslated region sequences for stabilizing nucleic acids by the method of claim 19;
(b) selecting the untranslated region sequences for stabilizing nucleic acids by the method of claim 20; and
(c) manufacturing the artificial nucleic acid so as to include a sequence having at least 70% homology with the untranslated region sequence selected in step (a) or (b).

22. A method for manufacturing an artificial nucleic acid comprising:
(a) selecting the untranslated region sequences for stabilizing nucleic acids by the method of claim 19;
(b) selecting the untranslated region sequences for stabilizing nucleic acids by the method of claim 20; and
(c) manufacturing the artificial nucleic acid so as to include sequences having at least 70% homology with the untranslated region sequences selected in steps (a) and (b).
